# EUROPÄISCHE PATENTSCHRIFT

(11) **EP 1 263 980 B1**
(45) Veröffentlichungstag und Bekanntmachung des Hinweises auf die Patenterteilung: **07.05.2008**
(21) Anmeldenummer: 01903766.2
(22) Anmeldetag: 13.02.2001
(51) Int. Cl.: C12P 19/04, A61L 17/00, A61L 27/20, A61L 29/04, C08L 1/02

(54) **VERFAHREN UND VORRICHTUNG ZUR HERSTELLUNG VON GEFORMTER MIKROBIELLER CELLULOSE ZUR VERWENDUNG ALS BIOMATERIAL, INSBESONDERE FUR DIE MIKROCHIRURGIE**
METHOD AND DEVICE FOR PRODUCING SHAPED MICROBIAL CELLULOSE FOR USE AS BIOMATERIAL, ESPECIALLY FOR MICROSURGERY
PROCEDE ET DISPOSITIF DE PRODUCTION DE CELLULOSE MICROBIENNE MOULEE DESTINEE A SERVIR DE BIOMATERIAU, NOTAMMENT POUR LA MICROCHIRURGIE

(30) Priorität: 17.02.2000 DE 10007798
(43) Veröffentlichungstag der Anmeldung: 11.12.2002
(73) Patentinhaber: Jenpolymers Ltd., 07745 Jena (DE)
(72) Erfinder: KLEMM, Dieter, 99425 Weimar (DE); MARSCH, Silvia, 04626 Stolzenberg (DE); SCHUMANN, Dieter, 07749 Jena (DE); UDHARDT, Ulrike, 07751 Golmsdorf (DE)
(74) Vertreter: Bock, Gerhard
(86) Internationale Anmeldenummer: PCT/EP2001/001621
(87) Internationale Veröffentlichungsnummer: WO 2001/061026

(56) Entgegenhaltungen:
- EP-A- 0 186 495
- EP-A- 0 396 344
- DATABASE CHEMABS [Online] CHEMICAL ABSTRACTS SERVICE, COLUMBUS, OHIO, US; KLEMM, D. ET AL: "Cellulose. BASYC, bacterially synthesized cellulose. Miniaturized tubes for microsurgery" retrieved from STN Database accession no. 132:69291 XP002166034 & POLYM. NEWS (1999), 24(11), 377-378 ,
- DATABASE CHEMABS [Online] CHEMICAL ABSTRACTS SERVICE, COLUMBUS, OHIO, US; YAMANAKA, SHIGERU ET AL: "Hollow microbial cellulose tubes for medical and other use" retrieved from STN Database accession no. 125:123795 XP002166035 in der Anmeldung erwähnt & JP 08 126697 A (AJINOMOTO KK, JAPAN) 21. Mai 1996 (1996-05-21)

## Beschreibung

Die Erfindung betrifft ein Verfahren und eine Vorrichtung zur Herstellung von geformter mikrobieller Cellulose zur Verwendung als Biomaterial, insbesondere für mikrochirurgische Anwendungen, beispielsweise als Ersatz für Blutgefäße und andere innere Hohlorgane oder als Manschette (cuff) zur Umhüllung von Nervenfasern u.ä.

Es ist bereits bekannt (z. B. JP 3 165 774 A1), mikrobielle Cellulose als Biomaterial für chirurgische Anwendungen, wie Gewebeitnplantate, beispielsweise für Bauchwand, Haut, subkutanes Gewebe, Organe, Verdauungstrakt, Speise-, Luft- und Harnröhre sowie Knorpel und Fettgewebe, einzusetzen.
Es ist ebenfalls bekannt (beispielsweise JP 8 126 697 A2, EP 186 495 A2, JP 63 205 109 A1, JP 3 165 774 A1), daß die mikrobielle Cellulose verwendungsspezifisch im Herstellungsprozeß geformt werden kann, beispielsweise als Blatt-, Stab-, Zylinder- und Bandform etc.
Folgende Methoden der Herstellung werden beschrieben:
- An der Oberfläche einer mit Cellulose-produzierenden Mikroorganismen beimpften Kulturlösung wird eine Platte fixiert und die Kultivierung durchgeführt. Es entsteht ein hohler Cellulosezylinder, dessen Querschnitt der Oberfläche der Nährlösung entspricht, die Kontakt zur Luft hat.
- Geformte mikrobielle Cellulose wird an gaspermeablen Materialien (synthetische oder natürliche Polymere) aufgebaut, indem die eine Seite des Materials mit einem Sauerstoff-enthaltenden Gas in Kontakt steht und die andere Seite mit der Nährlösung, so daß die mikrobielle Cellulose an dieser Seite gebildet und anschließend isoliert wird.
- Komplexe Hohlfasermembranen werden z. B. durch Beschichtung poröser Oberflächen (polymere Verbindungen) mit mikrobieller Cellulose erreicht, indem Kulturlösung in den äußeren (oder inneren) Raum einer Separationsmembran gegeben wird. Dann wird Luft durch den inneren (oder äußeren) Raum der Hohlfaser geleitet und eine komplexe Membran aufgebaut.
Diese Methoden sind mit folgenden Nachteilen für die Qualität der inneren Oberfläche des aufgebauten Hohlkörpers verbunden:
- Austrocknung
- Aufbau einer inhomogenen Celluloseschicht im Inneren des Hohlzylinders mit der Gefahr der Ablösung von Teilen der Cellulose (für Blutgefäßersatz, insbesondere im Mikrobereich, nicht verwendbar)
- Aufbau komplexer, nicht nur aus Cellulose bestehender Produkte (Beeinflussung der Bioverträglichkeit).
Es ist weiterhin bekannt (z. B. JP 3 272 772 A2), das geformte Biomaterial als kleinlumigen Blutgefäßersatz zu verwenden, wobei die Gefäßprothese an einem Hohlträger kultiviert wird, der Sauerstoffpermeabel ist (z. B. Cellophan, Teflon, Silicon, Keramik, ungewebtes Gewebe, Fasern).
Nachteilig ist, daß die auf diese Weise hergestellten Hohlzylinder keine hinreichend glatte Innenflächen aufweisen, wodurch sich in der eingesetzten Blutgefäßprothese Thromben ansiedeln können. Die Oberflächenqualität dieser Innenflächen ist um so entscheidender, je geringer der Durchmesser des Gefäßersatzes ist, da sich besonders kleinlumige Gefäße mit abgelagerten Thromben leicht verschließen können. In der Mikrochirurgie mit Gefäßdurchmessern von 1-3 mm oder geringer ist der Einsatz dieser Prothesen deshalb äußerst problematisch, wenn nicht gar unmöglich.
In der EP 396 344 A3 sind eine Hohlcellulose, die durch einen Mikroorganismus produziert wird, ein Prozeß zur Herstellung dieser Cellulose sowie ein künstliches aus dieser Cellulose geformtes Blutgefäß beschrieben.
Der erste Prozeß zur Herstellung der hohlen mikobiellen Cellulose schließt die Kultivierung eines Cellulose-produzierenden Mikroorganismus an der inneren und/oder äußeren Oberfläche eines Sauerstoff-permeablen Hohlträgers aus Cellophan, Teflon, Silikon, Keramik oder einem nicht-gewebten bzw. einem gewebten Material ein. Dieser Sauerstoff-permeable Hohlträger wird in eine Kulturlösung eingetaucht. Ein Cellulose-produzierender Mikroorganismus und ein Kulturmedium werden der inneren und/oder äußeren Seite des Hohlträgers zugeführt. Die Kultivierung erfolgt unter Zuführung eines sauerstoffhaltigen Gases (oder Flüssigkeit) ebenfalls an die besagte innere und/oder äußere Seite des Hohlträgers. Es bildet sich eine gallertartige Cellulose mit einer Dicke von 0,01 bis 20 mm an der Oberfläche des Hohlträgers. Auf Grund der Wechselwirkung des Cellulose-produzierenden Mikroorganismus, der produzierten Cellulose und des Hohlträgers entsteht ein Komposit von Cellulose und Hohlträger. Ist die Cellulose nicht an den Träger gebunden, wird dieser nach der Synthese der Cellulose entfernt und ein hohl geformter Artikel, der ausschließlich aus Cellulose besteht, kann erhalten werden.

Die so hergestellte Cellulose wird von Zellen des Mikroorganismus oder von Kulturlösungsbestandteilen mit verdünntem Alkali, verdünnter Säure, einem organischen Lösungsmittel und heißem Wasser, allein oder in Kombination davon gereinigt.

Der Nachteil dieser Methode besteht wiederum im Aufbau einer inhomogenen Celluloseschicht im Inneren des Hohlzylinders mit der Gefahr der Ablösung von Teilen der Cellulose (für Blutgefäße, insbesondere im Mikrobereich, problematisch).

Als zweiter Prozeß zum Aufbau von hohler mikrobieller Cellulose sind in der EP 396 344 A3 die Imprägnierung, ein ggf. erforderliches Nachbehandeln und das Schneiden der von einem Mikroorganismus produzierten Cellulose beschrieben. Ein mit Kulturlösung gefülltes Gefäß wird mit dem Mikroorganismus beimpft. Die aufgebaute mikrobielle Cellulose wird mit einem Medium, imprägniert und, sofern erforderlich, nachbehandelt, gefroren oder verdichtet. Dadurch wird die flüssige Komponente zwischen den die mikrobielle Cellulose bildenden Fasern zurückgehalten, um eine freie Bewegung der flüssigen Komponente zu verhindern. Danach wird der Schneideprozeß durchgeführt. Als Medium können Polyole, wie Glyzerol, Erythrol, Glycol, Sorbitol und Maltitol, Saccharide, wie Glucose, Galaktose, Mannose, Maltose und Lactose, natürliche und synthetische polymere Substanzen, wie Polyvinylalkohol, Polyvinylpyrrolidon, Polyethylenglycol, Carboxymethylcellulose, Agar, Stärke, Alginsäuresalze, Xanthan, Polysaccharide, Oligosaccharide, Kollagen, Gelantine, und Proteine, sowie wasserlösliche polare Lösungsmittel, wie Acetonitril, Dioxan, Essigsäure und Propionsäure, allein oder in Mischung verwendet werden.
Diese Methode ist mit folgenden Nachteilen in Hinsicht auf den Herstellungsaufwand und auf die Qualität der inneren Oberfläche des aufgebauten Hohlkörpers verbunden:
- keine direkte Formgebung während der Biosynthese
- hydrophile Eigenschaften der mikrobiellen Cellulose, die beispielsweise die Rauhigkeit der inneren Oberfläche sowie die Bioverträglichkeit bestimmen, sind verändert.
Als dritter Prozeß zur Herstellung einer hohlen mikrobiellen Cellulose wird in der EP 396 344 A3 die Herstellung mittels zweier Glasröhren unterschiedlicher Durchmesser beschrieben. Die Glasröhren werden ineinandergefügt, und im Raum zwischen den beiden Röhrenwandungen wird die Kultivierung des Mikroorganismus innerhalb von 30 Tagen durchgeführt. Es entsteht eine mikrobielle Cellulose mit hohlzylindrischer Gestalt, welche auf Grund einer guten Verträglichkeit mit dem lebenden Organismus, speziell mit Blut, als Blutgefäßersatz im lebenden Körper verwendet werden kann. Die Blutverträglichkeit (antithrombogene Eigenschaft) wurde beurteilt durch den Blutgefäßersatztest unter Verwendung eines erwachsenen Mischlingshundes. Es wurden Teile der absteigenden Aorta und Jugularvene des Hundes durch das künstliche Blutgefäß mit einem inneren Durchmesser von 2-3 mm ersetzt. Nach einem Monat wurde das künstliche Blutgefäß entnommen und der Zustand der Adhäsion von Thromben wurde untersucht. Eine leichte Ablagerung von Thromben ergab sich im Nahtbereich und eine nicht unwesentliche Adhäsion von Thromben wurde über die gesamte innere Oberfläche des künstlichen Blutgefäßes beobachtet (vgl. Beispiel 10 der Beschreibung). Es wird eine biologisch relativ gut verträgliche hohlzylindrische Cellulose geschaffen, die insbesondere als Ersatz für Blutgefäße mit einem Durchmesser von kleiner als 6 mm dienen kann. Allerdings ist der Einsatz durch die Gefahr von Thrombenablagerungen für kleinlumige Gefäße (im beschriebenen Beispiel 2-3 mm Durchmesser) nicht unbedenklich. Mikrochirurgische Anwendungen erfordern zudem noch kleinere Gefäßdurchmesser von 1 mm und darunter. Hier erscheint ein Einsatz dieser Gefäßprothesen auf Grund der genannten Thrombenadhäsion an ihrer Innenwand als nicht möglich.

Der Erfindung liegt deshalb die Aufgabe zu Grunde, ein Herstellungsverfahren für geformte Biomaterialien, insbesondere für mikrochirurgische Anwendungen als Ersatz von Blutgefäßen mit 1-3 mm Durchmesser und kleiner, anzugeben, das eine sehr hohe und reproduzierbare Qualität der in Blutkontakt tretenden Prothesenmaterialflächen gewährleistet und eine Thrombenadhäsion an diesen Flächen zuverlässig vermeidet
Die Biomaterialien sollen gewebe- und blutverträglich sowie, einschließlich der Fertigungszeit, möglichst aufwandgering und auch in beliebiger Form, insbesondere auch in variabler hohlzylindrischer Formgebung, herstellbar sein.
Die Aufgabe wird durch die kennzeichnenden Merkmale von Anspruch 1 und 5 erfüllt. Vorteilhafte Ausgestaltungen sind Gegenstand der jeweils nachgeordneten Ansprüche.
Die Nährlösung wird bekannter Weise sterilisiert, mit Cellulosebildenden Bakterien, beispielsweise mit einem eine formstabile Celluloseschicht produzierenden Stamm des Mikroorganismus *Acetobacter xylinum,* beimpft sowie in einem Raum zwischen Formkörperwandungen, beispielsweise bei einer Temperatur zwischen 28°C und 30°C kultiviert. Das bei der Kultivierung entstehende Biomaterial (Cellulose) wird von den Formkörperwandungen isoliert sowie einer Reinigung unterzogen (vgl. EP 396 344 A3).
Erfindungsgemäß wird die beimpfte Nährlösung jedoch nicht in den Zwischenraum zwischen den Formkörperwandungen, beispielsweise einer Glasmatrix aus vorzugsweise voneinander lösbaren Glaskörpern, eingefüllt, sondern die Formkörperwandungen (Glasmatrix) werden während der Kultivierung in ein Gefäß mit der beimpften Nährlösung eingetaucht, so daß die Nährlösung in den Zwischenraum zwischen den Formkörperwandungen durch Kapillarkraft eingezogen wird. Dadurch wird im Gefäß während des gesamten Kultivierungsprozesses ein feuchtes, aerobes Milieu zur Cellulosebildung gewährleistet.
Zur Herstellung hohlzylindrischer Cellulose als Blutgefäßersatz taucht eine an sich bekannte Glasmatrix, bestehend aus einem äußeren Glasrohr und einem axialsymmetrisch in diesem fixierten Glaskörper, in die beimpfte Nährlösung ein, die sich in dem besagten Gefäß, beispielsweise ein Erlenmeyerkolben, befindet. Nach der Kultivierung wird die Glasmatrix aus dem Gefäß herausgenommen und zur Entnahme der hergestellten Cellulose demontiert.
Als Formkörperwandung zur Formung der bei Einsatz des Biomaterials in Blutkontakt tretenden Prothesenmaterialfläche wird in jedem Kultivierungsprozeß jeweils ein ungebrauchter Formkörper hoher Oberflächengüte verwendet. Damit werden selbst mikroskopisch kleine Ablagerungen von Nährlösungsbestandteilen und ggf. Cellulosefasern an der Formkörperwandung zuverlässig ausgeschlossen, die im Fall einer Wiederverwendung der Formkörper trotz gründlichster Reinigung eine Veränderung der Haftungsbedingungen der sich bildenden Cellulose an der Formkörperwandung bewirken können. Bezogen auf die zylindrische Glasmatrix heißt das, daß für jeden neuen Kultivierungsprozeß ein ungebrauchter und die Innenwand des herzustellenden Gefäßersatzes formender Glaskörper im äußeren Glasrohr zu fixieren ist. Als zylindrische Glaskörper können zweckmäßig kommerziell erhältliche Schmelzpunktröhrchen mit Standardmaßen eingesetzt werden.
Überraschend hat sich mit diesen Verfahrensschritten gezeigt, daß in einem zu dem in der EP 396 344 A3 beschriebenen Anwendungsbeispiel relevanten Zeitraum keine vergleichbare Thrombenablagerung festgestellt werden konnte. Die Oberflächenqualität der auf diese Weise erzeugten sowie bei Implantation in Blutkontakt tretenden Prothesenmaterialflächen ist reproduzierbar sehr hoch und die Gefahr einer Thrombenadhäsion ist sehr gering. Damit eignen sich die erfindungsgemäß hergestellten Biomaterialien sehr gut als beständiger Blutgefäßersatz für mikrochirurgische Anwendungen, insbesondere für Gefäßdurchmesser von 1-3 mm und geringer.
Weitere Vorteile des vorgeschlagenen Verfahrens sind kurze Kultivierungszeiten (bereits nach 7 bis 14 Tagen bildet sich eine formstabile Celluloseschicht in der Glasmatrix heraus) sowie eine gute Verteilung der Impfkultur im Medium durch die Beimpfung der flüssigen Nährlösung mit einer flüssigen Stammkultur ("flüssig-flüssig-Beimpfung").
Die mittels zylindrischer Glasmatrix hergestellten röhrenförmigen Biomaterialien sind nicht nur als Gefäßprothesen, sondern auch als Manschette (cuff) zur Umhüllung von Nervenfasern u. ä. sowie als Übungsmaterial, insbesondere für das Training mikrochirurgischer Techniken, verwendbar. Mit letztgenannter Anwendung kann die Anzahl von Versuchstieren vermindert werden. Das bisher verwendete Übungsmaterial besteht beispielsweise aus Gummi und vermag möglichst realistische Operationsbedingungen nur unvollständig nachzuempfinden. In den Unteransprüchen sind weitere vorteilhafte Ausgestaltungen der Erfindung aufgeführt. Angegeben ist auch eine zweckmäßige Vorrichtung zur Durchführung des Herstellungsverfahrens, bei welcher der innere und bei jedem Kultivierungsprozeß jeweils neu verwendete Glaszylinder der Glasmatrix mittels muffenartiger elastischer Ringe an den Zylinderenden lagestabil und leicht lösbar im äußeren Glasrohr fixiert wird. Auf diese Weise ist die Glasmatrix unter Wiederverwendbarkeit des äußeren Glasrohres und vorgenanntem Austausch des inneren Glaszylinders mit geringstem Zeit- und Handhabungsaufwand demontierbar, und die hergestellte hohlzylindrische Cellulose kann problemlos material- und oberflächenschonend isoliert werden. Die Nährlösungs- und Luftzirkulation zum bzw. vom Zwischenraum der Glasmatrix wird dabei durch Öffnungen des Glasrohres, die sich im Bereich zwischen den elastischen Ringen der Glasmatrix befinden, gewährleistet. Der Einsatz einer solchen Vorrichtung ist effizient, da beim nachfolgenden Kultivierungsprozeß lediglich der innere zylindrische Glaskörper auszutauschen ist und aufwendige Reinigungsschritte entfallen bzw. auf ein Minimum beschränkt sind.
Zur Erhöhung der Ausbeute des hergestellten Biomaterials können gleichzeitig mehrere Glasmatrizes zur besagten Kultivierung in das Gefäß mit der beimpften Nährlösung eintauchen.
Das Herstellungsverfahren ist nicht auf die hohlzylindrische Formgebung des Biomaterials und ebenfalls nicht auf mikrochirurgische Anwendungen beschränkt.

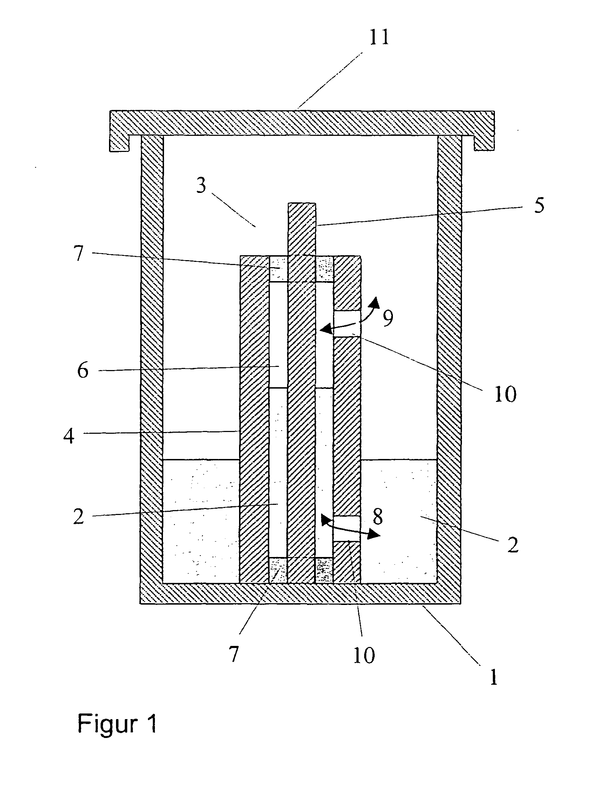

Die Erfindung soll nachstehend anhand eines in der Figur 1 dargestellten Ausführungsbeispiels näher erläutert werden.
Ein Gefäß 1 mit einer Kapazität von 50 ml wurde mit 20 ml einer Nährlösung 2 (Schramm-Hestrin-Medium), die pro Liter aqua dest. 20,00 g Glucose wasserfrei, 5,00 g Bactopepton, 5,00 g Hefeextrakt, 3,40 g di-Natriumhydrogenphosphat-Dihydrat und 1,15g Citronensäure-Monohydrat enthält sowie einen pH-Wert zwischen 6,0 und 6,3 aufweist, gefüllt. Die Nährlösung 2 wurde bei 120 °C für 20 Minuten dampfsterilisiert und danach mit dem Bakterium *Acetobacter xylinum* (AX 5, Stammsammlung des Institutes für Biotechnologie Leipzig) aus einer 10 Tage alten flüssigen Stammkultur (Schramm-Hestrin-Medium) beimpft. Im Anschluß daran wurde eine sterilisierte Glasmatrix 3, bestehend aus einem äußeren Glasrohr 4 und einem axialsymmetrisch darin fixierten inneren Glaskörper 5 mit einem Zylinderdurchmesser von 0,8 mm, in das Gefäß 1 eingetaucht. Auf Grund der wirkenden Kapillarkraft füllte sich ein Zwischenraum 6 zwischen dem äußeren Glasrohr 4 und dem inneren Glaskörper 5 mit der beimpften Nährlösung 2 des Gefäßes 1. Die Kultivierungszeit betrug 14 Tage bei einer Temperatur zwischen 28°C und 30 °C. In diesem Kultivierungszeitraum bildete sich eine weiße mikrobielle Cellulose sowohl im Gefäß 1 als auch im Zwischenraum 6 der Glasmatrix 3.

Die Glasmatrix 3 wurde aus dem Gefäß 1 entnommen und demontiert; die im Zwischenraum 6 der Glasmatrix 3 gebildete zylindrische mikrobielle Cellulose wurde isoliert, gründlich mit Wasser gewaschen, mit siedender, wässriger 0,1 N Natronlauge 10 Minuten lang behandelt und nochmals gründlich mit Wasser gewaschen, um eine Mila-ogefäßprothese mit einem inneren Durchmesser von 0,8 mm, einer Wandstärke von 0,7 mm und einer Länge bis zu 1 cm zu erhalten.

Die Blutverträglichkeit dieser Mikrogefäßprothese wurde beurteilt durch eine tierexperimentelle Studie, in der Teile der Arteria carotis von WISTAR-Ratten mit dem hergestellten künstlichen Blutgefäß ersetzt wurden. Dazu wurde vor der Operation das im gequollenen Cellulosematerial enthaltene Wasser gegen physiologische Kochsalzlösung ausgetauscht. Unmittelbar nach der Operation konnte ein ungehinderter Blutfluß beobachtet werden.

Nach einem Monat wurde das künstliche Blutgefäß entnommen, das durch die Einbettung in Bindegewebe und Ausbildung kleiner Blutgefäße innerhalb des Bindegewebes sehr gut in den tierischen Körper integriert und vollständig durchgängig war. Der Zustand der künstlichen Prothese, der Anastomosenbereiche und des Teils der Arteria carotis distal der zweiten Anastomose mit dem künstlichen Blutgefäß wurde histologisch und elektronemnikroskopisch untersucht. Es wurden keine Thrombenbildung und keine Proliferationsprozesse weder im Nahtbereich, im Interponat noch im Blutgefäß beobachtet.

Die Innenfläche der Prothese einschließlich Anastomosebereich wurde "biologisiert", das heißt vollständig mit Endothelzellen belegt (Ausbildung einer Neointima). Die innere Oberfläche der Anastomosen war flach und vollkommen unauffällig. Diese Ergebnisse wurden durch insgesamt 20 Tierversuche bestätigt.

Zur wiederholten Verwendung der Glasmatrix 3 in einem nachfolgenden Kultivierungsprozeß wurde der Glaskörper 5 gegen einen unbenutzten Glaskörper 5 ausgetauscht und der beschriebene Vorgang erneut durchgeführt.

Um den Glaskörper 5 im Glasrohr 4 mit möglichst geringem Handhabeaufwand lagestabil zu fixieren und die Glasmatrix 3 ebenso aufwandgering und vor allem materialschonend in Bezug auf die hergestellte Cellulose wieder demontieren zu können, wird der Glaskörper 5 mit muffenartigen Silikonringen 7 im Glasrohr 4 fixiert. Damit jedoch ein Nährlösungsaustausch 8 und eine weitgehend ungehinderte Luftzirkulation 9 gewährleistet wird, besitzt das Glasrohr 4 im Bereich zwischen den Silikonringen 7 Öffnungen 10. Zur Gewährleistung der Sterilität und eines feuchten, aeroben Milieus im Gefäß 1, wird dieses während des Kultivierungsprozesses mit einem Deckel 11 verschlossen.

### Bezugszeichenliste

| | | |
|---|---|---|
| 1 | - | Gefäß |
| 2 | - | Nährlösung |
| 3 | - | Glasmatrix |
| 4 | - | Glasrohr |
| 5 | - | Glaskörper |
| 6 | - | Zwischenraum |
| 7 | - | Silikonring |
| 8 | - | Nährlösungsaustausch |
| 9 | - | Luftzirkulation |
| 10 | - | Öffnung |
| 11 | - | Deckel |

## Patentansprüche

1. Verfahren zur Herstellung von geformter mikrobieller Cellulose zur Verwendung als Biomaterial, insbesondere für mikrochirurgische Anwendungen, bei dem eine sterilisierte Nährlösung mit Cellulosebildenden Bakterien, beispielsweise ein eine formstabile Celluloseschicht produzierender Stamm des Mikroorganismus *Acetobacter xylinum,* beimpft und die Bakterien in einem Raum zwischen Formkörperwandungen kultiviert werden und bei dem das bei der Kultivierung entstehende Biomaterial von den Formkörperwandungen isoliert sowie einer Reinigung unterzogen wird, **dadurch gekennzeichnet, daß** die Wandungen der Formkörper (4; 5) in einen so engen Abstand gebracht werden, daß die Nährlösung (2) zwischen ihnen durch Kapillarkraft angehoben wird und diese in ein Gefäß (1) mit der beimpften Nährlösung (2) eingetaucht werden, wobei der Mikroorganismus sowohl im Gefäß (1) als auch im Raum (6) zwischen den Formkörperwandungen zur Cellulosebildung in einem feuchten, aeroben Milieu kultiviert wird, wobei während des gesamten Kultivierungsprozesses ein Nährlösungs- und Sauerstoffaustausch über Öffnungen (8, 10) zwischen der Nährlösung im Zwischenraum (6) und im Gefäß (1), das während der Kultivierung durch Mittel (11) verschlossen wird, erfolgt, und daß als Formkörperwandung zur Formung der bei Einsatz des Biomaterials in Blutkontakt tretenden Prothesenmaterialfläche in jedem Herstellungsprozeß jeweils ein ungebrauchter Formkörper hoher Oberflächengüte, vergleichbar der von Glas, verwendet wird.

2. Verfahren nach Anspruch 1, **dadurch gekennzeichnet, daß** als Formkörperwandungen, zwischen denen der Mikroorganismus kultiviert wird, eine Glasmatrix aus voneinander lösbaren Glaskörpern verwendet wird.

3. Verfahren nach Anspruch 2, **dadurch gekennzeichnet, daß** zur Herstellung von hohlzylindrischem Biomaterial eine Glasmatrix, bestehend aus einem äußeren Glasrohr und einem axialsymmetrisch darin eingefügten Glaskörper geringeren Durchmessers, in das Gefäß mit der beimpften Nährlösung eingetaucht wird.

4. Verfahren nach Anspruch 2, **dadurch gekennzeichnet, daß** zur gleichzeitigen Herstellung mehrerer Biomaterialien mehrere Glasmatrizes in das Gefäß mit der beimpften Nährlösung eingetaucht werden.

5. Vorrichtung zur Durchführung des Verfahrens nach Anspruch 3, **dadurch gekennzeichnet, daß** mindestens eine Glasmatrix (3), bestehend aus einem äußeren Glasrohr (4) und einem axialsymmetrisch darin eingefügten Glaskörper (5) geringeren Durchmessers, in ein Gefäß (1) mit darin befindlicher beimpfter Nährlösung (2) eintaucht, wobei der innere Glaskörper (5) zum Zweck einer einfach handhabbaren, lagestabilen und leicht lösbaren axialsymmetrischen Zentrierung im Glasrohr (4) durch elastische Ringe (7) unter Gewährleistung eines Nährlösungsaustausches (8) und einer Luftzirkulation (9) in bzw. aus einem das herzustellende Biomaterial formenden Zwischenraum (6) der Glasmatrix (3) fixiert wird, wobei der die Glasmatrix (3) umgebende Raum Nährlösung (2) beinhaltet und mit einer Abdeckung (11) versehen ist.

6. Vorrichtung nach Anspruch 5, **dadurch gekennzeichnet, daß** der Nährlösungsaustausch (8) und die Luftzirkulation (9) durch jeweils mindestens eine Öffnung (10) des äußeren Glasrohres (4) innerhalb des Bereiches der Glasmatrix (3) zwischen den elastischen Ringen (7) gewährleistet wird.

7. Vorrichtung nach Anspruch 5, **dadurch gekennzeichnet, daß** als Gefäß (1), in welches die Glasmatrix (3) eintaucht, ein an sich bekannter Erlenmeyerkolben verwendet wird.

## Claims

1. Method for producing shaped microbial cellulose for application as biomaterial, in particular for microsurgical applications, in which a sterile culture medium is inoculated with cellulose generating bacteria, for example, with a strain of the microorganism *Acetobacter xylinum* generating a form-stable cellulose layer, and the bacteria are cultivated in a space between the walls of a shaping body and in which the biomaterial resulting from the cultivation is isolated from the walls of said shaping body as well as subjected to a cleaning procedure, **characterized in that** the walls of the shaping body (4; 5) are arranged at such a short distance from each other that the culture medium (2) between them is raised by capillarity and that these are immersed into a vessel (1) containing the inoculated culture medium (2), whereby the microorganism is cultivated for cellulose formation both in the vessel (1) and in the space (6) between the walls of the shaping body in a moist and aerobic environment, whereby during the entire cultivation process, via openings (8, 10), a culture medium exchange and an oxygen exchange take place between the culture medium in the interspace (6) and in the vessel (1) which is closed by means (11) during cultivation, and that in each inculturing process an always unused shaping body of high surface quality, comparable to that of glass, is used as a shaping body wall for shaping the prosthesis material surface which comes into contact with the blood when the biomaterial is applied.

2. Method as claimed in claim 1, **characterized in that** a glass matrix of glass bodies being detachable from each other is used for the shaping body walls between which the microorganism is cultivated.

3. Method as claimed in claim 2, **characterized in that** for producing hollow cylindrical biomaterial, a glass matrix, comprised of an outer glass tube and a glass body of smaller diameter inserted into said glass tube in axial symmetry is immersed into the vessel containing the inoculated culture medium.

4. Method as claimed in claim 2, **characterized in that** for the simultaneous production of a plurality of biomaterials, a plurality of glass matrices is inserted into the vessel containing the inoculated culture medium.

5. A device for carrying out the method as claimed in claim 3, **characterized in that** at least one glass matrix (3), comprised of an outer glass tube (4) and a glass body (5) of smaller diameter being inserted into said glass tube (4) in axial symmetry, is immersed into a vessel (1) containing the inoculated culture medium (2), whereby the inner glass body (5) is fixed by elastic rings (7) for the purpose of an easy manipulation and an easily detachable axisymmetric centering in stable position within said outer glass tube (4), under provision of a culture medium exchange (8) and an air circulation (9) into or out of an interspace (6) of the glass matrix (3), said interspace being for shaping said biomaterial to be produced, whereby the space surrounding the glass matrix (3) contains the culture medium (2) and is provided with a cover (11).

6. Device as claimed in claim 5, **characterized in that** the culture medium exchange (8) and the air circulation (9) are ensured by at least one respective opening (10) of the outer glass tube (4) within the range of the glass matrix (3) between the elastic rings (7).

7. Device as claimed in claim 5, **characterized in that** an Erlenmeyer flask, known per se, is used as the vessel (1) into which the glass matrix (3) is immersed.

## Revendications

1. Le Procédé de production de cellulose microbienne moulée destinée à servir de biomatériau, notamment pour les applications microchirurgiennes, au cours duquel une solution nutritive stérilisée est inoculée avec des bactéries produisant de la cellulose, par exemple sous forme de souche du microorganisme *Acetobacter xylinum* et susceptible de produire une couche cellulosique moulée stable ; les bactéries sont cultivées dans un espace situé entre les parois des corps moulés et le biomatériau produit en culture, isolé des parois des corps moulés, puis nettoyé, est **caractérisé en ce que** les parois des corps moulés (4 ; 5) sont disposées de manière si proches les unes des autres que la solution nutritive (2) se trouvant dans les interstices entre les parois se trouve soulevée par capillarité et immergée dans un récipient (1) comprenant la solution nutritive inoculée (2), le microorganisme étant cultivé en milieu humide anaérobie aussi bien dans le récipient (1) que dans l'espace (6) entre les parois des corps moulés pour produire de la cellulose, et que, durant le développement de la culture, se produit un échange de solution nutritive et d'oxygène à travers les orifices (8, 10) entre la solution nutritif se trouvant dans l'espace (6) et celle dans le récipient (1) étant obturé pendant le développement de la culture par des moyens appropriés (11), et que dans chaque processus de production est utilisé un corps moulé neuf d'une haute qualité surfacique, comparable à celle du verre, servant de paroi du corps moulé pour le formage de la surface de matériel prothétique en contact avec du sang d'un biomatériau.

2. Le procédé suivant la revendication 1 est **caractérisé en ce que** les parois des corps moulés, entre lesquelles le microorganisme est cultivé, sont constituées d'une matrice de verre composée de corps de verre non solidaires les uns par rapport aux autres.

3. Le procédé suivant la revendication 2 est **caractérisé en ce que**, pour produire des biomatériaux en forme de cylindres creux, il faut qu'une matrice de verre, composée d'un tube de verre extérieur et d'un corps de verre de faible diamètre intégré de manière axisymétrique dans ce tube de verre, soit immergé dans un récipient contenant la solution nutritive inoculée.

4. Le procédé suivant la revendication 2 est **caractérisé en ce que**, pour produire simultanément plusieurs biomatériaux il convient que plusieurs matrices de verre soit immergées dans le récipient contenant la solution nutritive inoculée.

5. Le dispositif destiné à assurer le procédé suivant la revendication 3 est **caractérisé en ce qu'**au moins une matrice de verre (3) composée d'un tube de verre extérieur (4) et d'un corps de verre (5) de faible diamètre y intégré de manière axisymétrique, soit immergée dans un récipient (1) contenant une solution nutritive (2), le corps de verre intérieur (5) étant fixé dans le tube de verre (4) par des anneaux élastiques (7) afin d'assurer son centrage axisymétrique en position stable, facilement maniable et détachable, en permettant un échange de solutions nutritives (8) et une circulation d'air (9) dans un espace intermédiaire (6) de la matrice de verre (3), formant le biomatériau à produire, l'espace entourant la matrice de verre (3) contenant la solution nutritive(2) étant doté d'une couverture (11).

6. Le dispositif suivant la revendication 5 est **caractérisé en ce qu'**au moins un orifice (10) dans le tube de verre extérieur (4) au sein de la zone de la matrice de verre (3) situé entre les anneaux élastiques (7) assure l'échange de solutions nutritives (8) et la circulation d'air (9).

7. Le dispositif suivant la revendication 5 est **caractérisé en ce que** le récipient (1), dans lequel la matrice de verre (3) soit immergée, est un vase d'Erlenmeyer tel que l'on connaît.
